# EUROPEAN PATENT SPECIFICATION

(11) **EP 1 368 419 B1**
(45) Date of publication and mention of the grant of the patent: **16.11.2005**
(21) Application number: 02718481.1
(22) Date of filing: 25.01.2002
(51) Int. Cl.: C08J 9/00

(54) **A METHOD OF PREPARING A COLLAGEN SPONGE, A DEVICE FOR EXTRACTING A PART OF A COLLAGEN FOAM, AND AN ELONGATED COLLAGEN SPONGE**
VERFAHREN ZUR HERSTELLUNG EINES KOLLAGEN-SCHWAMMES; EXTRAKTIONSVORRICHTUNG VON EINEM TEIL EINES KOLLAGEN-SCHWAMMES UND VERLÄNGERTER KOLLAGEN-SCHWAMM
PROCEDE PERMETTANT DE PREPARER UNE EPONGE COLLAGENIQUE, DISPOSITIF PERMETTANT D'EXTRAIRE UNE PARTIE DE MOUSSE COLLAGENIQUE, ET EPONGE COLLAGENIQUE ALLONGEE

(30) Priority: 25.01.2001 DK 200100135; 13.02.2001 DK 200100235
(43) Date of publication of application: 10.12.2003
(73) Proprietor: Nycomed Pharma AS, 1371 Asker (NO)
(72) Inventor: SCHAUFLER, Alfred, A-4048 Puchenau (AT)
(74) Representative: Plougmann & Vingtoft A/S
(86) International application number: PCT/IB2002/001452
(87) International publication number: WO 2002/070594

(56) References cited:
- EP-A- 0 578 823
- FR-A- 2 668 936
- GB-A- 1 292 326
- US-A- 4 626 286
- CHEMICAL ABSTRACTS, vol. 98, no. 24, 13 June 1983 (1983-06-13) Columbus, Ohio, US; abstract no. 204391, VIDAL B.C. ET AL.: "Microfibrous and microcrystalline collagen I for use in medicine and pharmacy" XP002170464 cited in the application & BR 8 102 435 A 30 November 1982 (1982-11-30)
- PATENT ABSTRACTS OF JAPAN vol. 1995, no. 6, 31 July 1995 (1995-07-31) & JP 07 059812 A (KOKEN CO LTD), 7 March 1995 (1995-03-07) & DATABASE WPI Week 199518 Derwent Publications Ltd., London, GB; AN 1995-135943

## Description

### TECHNICAL FIELD

The present invention relates to a method of preparing a collagen sponge. The collagen sponge produced according to the invention is in particular useful in surgery primarily to stop capillary bleeding. The collagen sponge may also be used as a carrier to be coated with a fibrin glue preparation. The invention also relates to a device for extracting a part of a collagen foam. The invention further relates to an elongated collagen sponge, primarily for gastrointestinal use.

### BACKGROUND OF THE INVENTION

Collagen has been used as a hemostyptic agent since the late sixties. Collagen is the most frequent structural protein in all mammalians. The monomeric protein of approximately 300 kDa (tropocollagen) is covalently crosslinked at specific sites. The mature protein is therefore insoluble and forms characteristic fibrils with high tensile strength. Numerous sub-classes of collagen have been described, the most common of which is collagen type I, the main collagen type in skin, tendons bones and cornea. Collagen is a fibrous protein consisting substantially of a triple helix with a length of approximately 290 nm. Five of these triple helices (tropocollagen molecules) are staggered to form a microfibril with a diameter of approximately 3.6 nm. These microfibrils have polar and non-polar segments that are readily accessible for specific inter- and intrafibrillar interactions. Microfibrils are packed into a tetragonal lattice to form subfibrils with a diameter of about 30 nm. These subfibrils are then assembled into the collagen fibril, the basic unit of connective tissue, which has a diameter of several hundred nm and is therefore visible in a light microscope as a thin line, see reference 1. Collagen gel and collagen sponge, as produced during the manufacturing process, comprises these fibrils as the smallest units, as proved by microscopy.

Collagen may be used as a material for sealing wounds, possibly with a coating comprising a fibrin glue. Fibrin glues i.e. the combination of fibrinogen, thrombin and aprotinin have successfully been used therapeutically for many years for gluing tissues and nerves and for sealing surfaces when there is minor bleeding. One draw back of the fibrin glues has been that in case of major bleeding the glue is usually washed away before sufficient polymerisation of fibrin has occurred. To overcome this problem surgeons have manually applied liquid fibrin glues to absorbable carriers such as collagen fleece.

Despite the impressive success of these combined applications this method has not been applied on a broad scale, due to some disadvantages. The preparation is relatively cumbersome, the method requires experience and skilled personnel, and the preparation is not readily available in cases of emergency, the time for preparation being in the range of 10 to 15 min. These factors stimulated the development of an improved product resulting in the development of a fixed combination of a collagen carrier covered with a coating of solid fibrinogen, solid thrombin and solid aprotinin as disclosed in EP 0 059 265. The product disclosed in EP 0 059 265 which has been marketed under the trademark TachoComb® can be applied directly to the wound. When the coating comes into contact with aqueous fluids like blood, other body fluids or saline, the components dissolve and fibrin is formed. The product is applied to the wound with a slight pressure and collagen is tightly bound (glued) to the injured surface. Haemostasis is achieved and the wound is sealed.

Beside some blood coagulation stimulating activity, the function of collagen in TachoComb® is mainly that of a carrier which adsorbs and confers mechanical stability to the coagulation preparation with which it is coated. Other advantages of collagen, in particular in the form of a sponge, are its biodegradability, its relatively high tensile strength, even in the wet state, its high resistance against the penetration of liquids and air, and its high flexibility in the wet state.

The present invention is primarily concerned with the production of a collagen sponge which may be used as a carrier for fibrinogen, thrombin and/or aprotinin, e.g., as in TachoComb®. The collagen sponge may also be used directly, i.e. without a coating, as a bandage on topical injuries, for support of haemostasis, such as for prevention of rebleeding, for weak, diffuse bleeding from parenchymatic organs, for application on bums, skin grafts, decubitus or skin defects, or as a bandage on topical injuries.

In the prior art, a number of methods for preparing a collagen carrier have been suggested. WO 86/05811 discloses a weighted microsponge for immobilizing bioactive materials in motive bioreactor systems, the microsponge comprising a highly cross-linked collagen matrix. The highly cross-linked collagen matrix is prepared by milling a source of Type I, II or III collagen to yield fibers having a diameter on the order of 1 to 50 µm and a length no greater than 200 µm. The milled collagen is formed into a soluble collagen dissolved in a solvent, or an insoluble collagen dispersed in a solvent by admixture with a solvent, such as acetic acid, lactic acid, proprionic acid or butyric acid. In the case of a collagen dispersion, the mixing is accomplished with a high level of agitation using a blender, so as to produce microfibers of the collagen. Next, a weighting additive is blended with the collagen-liquid mixture and the composite mixture is formed into small droplets and solidified by freezing. A number of techniques for producing small particles are disclosed. The frozen composite is vacuum freeze-dried, the combination of freezing and drying being referred to as lyophilization. The freeze-dried collagen matrix composite is treated so as to cross-link the collagen. The collagen can be cross-linked using either chemical cross-linking agents, by severe dehydration at an elevated temperature or by a combination. The collagen matrix aimed at being resistant to collagenase and other enzymatic degradation thereby making these materials particularly suitable for culturing organisms. After washing the cross-linked collagen matrix, the microsponges may be sterilized and aseptically packaged. In the weighted microsponge, the collagen matrix has an open to the surface pore structure with an average pore size in the range of from about 1 to about 150 µm, the pores of the matrix occupying from about 70 to about 98% by volume of the microsponge. The microsponge further has an average particle size of from about 100 to about 1000 µm and a specific gravity of above about 1.05. The weighting material may be metal or alloys from metal, metal oxides and ceramics.

US 5,660,857 discloses a process for preparing a composite comprising an insoluble protein matrix and an oleaginous material, which is useful as a material for surgical dressings and biomedical implants, and as a cosmetic material for application to the skin. The process of US 5,660,857 comprises the steps of mixing a protein, the oleaginous material and water to form an emulsion of the oleaginous material in an aqueous dispersion of the protein, and subsequently drying or freeze-drying the emulsion to form a film or a sponge. The insoluble fibrous protein is predominantly comprised of insoluble collagen, which may suitably be obtained from bovine skin. In one embodiment, the collagen may be swollen in lactic acid prior to use.

WO 99/13902 discloses a method for producing a meningeal tissue growth matrix comprising the step of preparing physiologically compatible collagen which is substantially free of active viruses and prions. The collagen is formed into a film, a sponge, a non-woven collagen or a felt. The collagen is obtained by a process comprising cleaning skin, tendons, ligaments or bone of fat. The material is then subjected to an enzyme treatment, whereby the collagen material is swelled. The collagen material is then further swollen with an acid solution. The collagen mixture is then homogenised. The product obtained may be a matrix provided in the form of a collagen sponge, a non-woven matrix, felt or film, or a composite of two or more of he foregoing forms. A collagen sponge can be provided by adaptation of the methods for forming collagen sponges disclosed in US 5,019,087. The sponge can be prepared by lyophilization of a collagen dispersion prepared according to WO 99/13902. The sponge density achieved is said to be about 0.1 mg/cm³ to about 120 mg/cm³. According to the disclosure of WO 99/13902, the pore size ranges from about 10 µm to about 500 µm. Laminate type of collagen sponge and collagen film are mentioned.

US 5,618,551 relates to a non-crosslinked and potentially crosslinkable pepsintreated collagen or gelatin powder modified by oxidative cleavage in an aqueous solution, which is soluble at an acid pH and stable on storage at a temperature of below 0°C for at least one month. The patent further relates to a process of preparing the powder, comprising preparing an acidic solution of pepsin-treated collagen, subjecting the acidic aqueous solution at room temperature to controlled oxidation, precipitating the oxidized and noncrosslinked pepsintreated collagen at an acid pH, and isolating, concentrating and dehydrating the noncrosslinked pepsintreated collaged so as to obtain it in the form of a reactive acidic powder, and freezing and storing the obtained reactive acidic powder at a temperature of below 0°C.

GB 1 292 326 discloses a method and apparatus for the preparation of collagen dispersions with a view to their applications, wherein a suspension of collagen fibres is prepared and subsequently introduced into a treatment chamber with stirring means. A sub-atmospheric pressure exists in the treatment chamber, in which the suspension is transformed into a dispersion by stirring and controlled acidification by means of a mineral or organic acid. According to the disclosure of GB 1 292 326, the preparation of spongy collagenic articles can be effected from dispersion or gels of collagen. In this context the documents refers to lyophilization and to dispersion or gels very rich in air bubbles. GB 1 292 326 further mentions a problem of controlling the introduction or the elimination of air bubbles in a satisfactory manner. The documents discloses, in two examples, a collagenic dispersion free of air bubbles with a collagen content of 2.5%, and an aerated dispersion of collagen with a collagen concentration of 2.5%, respectively.

Chemical Abstracts, Columbus Ohio, US, Vol. 98 13 June 1983 No. 24 mentions a collagen obtained from animal tissues such as skin or tendon bone which has been submitted to acid treatment. The collagen is reaggregated by dialysis, during which process a net of highly birefringent crystal fibres is formed. The collagen can be shaped into 0.5 mm - 2 cm sheets, or be mixed with air to form sponges, or be dispersed as a cream.

### DESCRIPTION OF THE INVENTION

It has been found that the successful coating of a collagen sponge with a fibrin glue preparation depends on the texture of the collagen sponge. It is thus an object of the present invention to provide a method of producing a collagen sponge with a certain texture, in particular with the aim of making the collagen sponge suitable for coating with a fibrin glue preparation, so as to obtain a material for healing and sealing wounds. It is a further object of the invention to provide a method of producing a collagen sponge having improved physical characteristics in relation to prior art sponges, in the sense of improved humidity, elasticity, density and elasticity module. It is a further object of the invention to provide a method for preparing a collagen sponge which is air and liquid tight in the sense that, once the collagen sponge is applied to a wound, it will not allow air or liquid to soak through the collagen sponge. It is a still further object of the invention to provide a wound closing material which can be used in gastrointestinal funnels or trachea.

Thus, in a first aspect the invention provides a method of preparing a collagen sponge, comprising the steps of:
- preparing a collagen gel,
- mixing air into the Collagen gel, so as to obtain a collagen foam,
- drying the collagen foam, so as to obtain a dry block of collagen sponge having chambers therein,
- isolating, from the block of collagen sponge, parts of sponge with a chamber diameter of more than 0.75 mm and less than 4 mm, or having a chamber diameter average of at most 3 mm.

In the present context, the term "chamber diameter" should be understood as the largest straight-line wall-to-wall distance in a chamber, i.e. as the largest diagonal straight-line distance of a chamber. The chambers may be of a polygonal shape, such as of an octagonal shape.

It has been found that a chamber diameter of more than 0.75 mm and less than 4 mm, or a chamber diameter average of at most 3 mm, renders the collagen sponge particularly useful for being coated with a fibrin glue preparation. Preferably, the Collagen gel has a dry mass in the range of 2-20 mg dry mass per 1 g gel, such as 4-18 mg, such as 5-13 mg, such as 6-11 mg per 1 g gel. The dynamic viscosity of the collagen gel is preferably 2-20 Ncm, such as 4-10 Ncm, such as 6-8 Ncm. The Collagen sponge preferably has a water content of not more than 20%, such as 10-15%, such as about 18%. The elasticity module of the collagen sponge is preferably in the range of 5-100 N/cm², such as 10-50 N/cm², and the density of the sponge is preferably 1-10 mg/cm³, such as 2-7 mg/cm³.

It has been found that a collagen sponge prepared by the method according to the invention is air and liquid tight in the sense that, once the collagen sponge is applied to a wound, it will not allow air or liquid to pass through the collagen sponge. Liquids are absorbed in the sponge. This effect is primarily achieved due to the fact that the step of mixing air into the collagen gel provides a collagen sponge which has a three-dimensional structure with stacked chambers separated and substantially totally enclosed by walls of collagen material, in contradiction to those known collagen sponges which have a fibre structure.

The collagen gel may comprise material of different types, such as type I, II or III from mammalian, transgenic or recombinant sources, but all other types of collagen can be used. The collagen may comprise material from tendons selected from the group consisting of equine tendons, human tendons, and bovine tendons. The collagen gel may additionally or alternatively comprise recombinant collagen material.

The collagen content of the isolated parts of sponge is preferably 50% - 100% related to dry mass of the sponge, such as 75% - 100%, such as 80% - 100%, such as 85% - 100%, such as 90% - 100%, such as 92 - 100%, such as 92 - 98%, such as 93 - 97%, such as 94% - 96%.

The step of preparing the collagen gel preferably comprises the steps of:
- storing the tendons at a temperature between -10°C and -30°C, and peeling the tendons,
- removing foreign protein from the tendons,
- reducing germ content in the tendons,
- swelling the tendons,
- homogenising the swelled tendons.

The steps of storing, peeling, removing protein, reducing of germ content, and swelling aim at purifying the raw material, whereas the step of homogenising aims at obtaining the collagen in the form of a gel.

The step of reducing of germ content preferably comprises adding an acid, such as an organic acid, such as lactic acid to the tendons. Further, an organic solvent, such as an alcohol, such as ethanol is preferably added to the tendons. Further, the step of swelling of the tendons preferably comprises adding lactic acid to the tendons. The lactic acid used may be a 0.40 - 0.50% lactic acid, such as a 0.45% lactic acid.

The step of swelling of the tendons may comprise storing the tendons at a temperature of 4°C to 25°C, such as a temperature of 10°C to 20°C, for a period of 48 to 200 hours, such as a period of 100 to 200 hours.

The step of homogenising the swelled tendons is preferably carried out so as to obtain a particle size of collagen gel fragments, i.e. fibre balls, with a diameter of 0.8 - 1.2 cm, such as approximately 1 cm. Further, the physical characteristics of the collagen gel are preferably as stated above. The appropriate characteristics may for example be achieved by performing the step of homogenising the swelled tendons by means of a toothed disk mill or adequate homogenisation equipment.

The step of mixing air into the collagen gel preferably comprises the steps of:
- mixing ambient air into the gel by means of a mixer so as to generate a collagen foam,
- feeding the mixed gel foam into a fractionising channel,
- separating collagen gel and collagen foam contained in the fractionising channel.

At least some of the collagen gel separated from the collagen foam in the fractionising channel may be led back to the mixer. In that case, the ratio between the amount of collagen gel which is led back to the mixer from the fractionising channel and the amount of fresh collagen gel led to the mixer is preferably between 0.1 and 0.5. The step of separating collagen gel and collagen foam preferably comprises the steps of:
- separating a selected part of the collagen foam contained in the fractionising channel,
- leading the selected part of the collagen foam out of the fractionising channel for drying thereof.

In a preferred embodiment of the method, a temperature of 15°C to 40°C, such as 20°C to 25°C is maintained in the fractionising channel.

Subsequent to mixing air into the collagen gel, the collagen foam may be homogenised for a period of 2 to 4 minutes.

Prior to the step of drying the collagen foam and subsequent to the step of mixing air into the collagen gel, a neutraliser may be added to the collagen foam, and the collagen foam is preferably neutralised in order to arrive from a pH-value of, usually, between 2.5 and 3.5 to a pH-value in the collagen foam between 6.5 and 8.5. A neutraliser comprising an ammonia solution may be used, and the collagen foam is preferably neutralised for a period of 5-30 hours, such as 10-20 hours, such as approximately 24 hours.

Prior to the step of drying the collagen foam, the collagen foam is preferably filled into a drying container in such a way that substantially no air is drawn into the foam while filling.

The step of drying preferably comprises drying at a temperature between 15°C and 60°C, such as between 20° and 40°C, for a period of 50-200 hours, such as 100-150 hours, so as to obtain a dry collagen sponge. The drying may be performed at a pressure slightly under atmospheric pressure, such as at a pressure of between 700 and 900 mbar, such as approximately 800 mbar.

The collagen sponge produced by the method according to the invention preferably fulfils at least one of the following criteria:
- pH-value between 5.0 and 6.0,
- lactic acid content at the most 5%,
- ammonium content at the most 0.5%,
- soluble protein content, calculated as albumin content, at the most 0.5%,
- sulphate ashes content at the most 1.0%,
- heavy metal content at the most 20 ppm,
- microbiological purity, at the most 10³ CFU/g,
- collagen content of 75% to 100%,
- density of 1-10 mg/cm³, such as 2-7 mg/cm³,
- elasticity module of 5-100 N/cm², such as 10-50 N/cm².

The step of isolating parts of collagen sponge may comprise dividing the collagen sponge into a plurality of parts by cutting. The parts obtained may be shaped in any desirable form, such as conical, cylindrical, including cylindrical with an annular cross-section, rectangular, polygonal, cubic, and flat sheets or they may be transformed into a granulate by an appropriate granulating method etc.

In a second aspect, the present invention relates to a method of preparing a collagen sponge, comprising the steps of:
- preparing a collagen gel,
- mixing air into the collagen gel, so as to obtain a collagen foam,
- drying the collagen foam, so as to obtain a dry block of collagen sponge having chambers therein,
- isolating, from the block of collagen sponge, parts of sponge having the following properties:
   - elasticity module in the range of 5 to 100 N/cm,
   - density in the range of 1 to 10 mg/cm³,
   - chamber diameter of more than 0.75 mm and less than 4 mm, or a chamber diameter average of at most 3 mm.

It should be understood that any and all steps of the method according to the first aspect of the invention may also be performed in the method according to the second aspect of the invention. Further, any and all characteristics and features of the collagen sponge produced by the method according to the first aspect of the invention may also be achieved by the method according to the second aspect of the invention.

According to the present invention a device may be used for extracting a part of a collagen foam and for degenerating another part of the collagen foam to a collagen gel, the device comprising:
- a fractionising channel comprising an inlet for receiving a flow of collagen foam, an outlet for a part of the flow of collagen foam, and a bottom portion which is inclined downwards in the direction of the flow of collagen foam,
- at least one outlet for collagen gel at the bottom portion of the fractionising channel, wherein the position of the outlet is movable in a vertical direction at an end of the fractionising channel.

The method of, the present invention may provide an elongated collagen sponge having a through-going hole or bore and a flexible wall. In a preferred embodiment, such a collagen sponge may be used closing wounds or re-establishing gastrointestinal funnel and trachea walls in mammalians. Thus, the collagen sponge may have circular or elliptical cross-section. The collagen sponge may be applied both as a filling, or as in a gastrointestinal funnel, or as an outer sleeve applied to an outer surface of a gastrointestinal funnel.

The inner diameter of the through-going hole or bore may, for application in various human gastrointestinal funnels and trachea, for example be as follows:

| | |
|---|---|
| Bowels | 0.5 - 6 cm |
| Rectum | 1 - 4 cm |
| Large intestine | 2 - 6 cm |
| Small intestine | 0.5 - 3 cm |
| Oesophagus | 0.5 - 2 cm |
| Trachea | 1 - 4 cm |

The collagen sponge may, e.g., be used for closing wounds after surgical removal of outpouchings on gastrointestinal funnel walls, such as after rectal surgery, such as after surgical removal of hemorrhoids. Examples of indications made possible by the collagen sponge according to the invention are:
- wound dressing,
- support of haemostasis, such as
   - weak, diffuse bleeding from parenchymatic organs,
   - surgical procedures on surgery locations where ectrosurgery or ligation has been performed prior to application of the collagen sponge,
   - prevention of rebleeding (securing of sutures),
   - application on burns,
   - bandage on topical injuries,
- drug delivery, such as delivery of antibiotics.

### BRIEF DESCRIPTION OF THE DRAWINGS

Figs. 1 and 2 contain a flow chart illustrating the steps involved in a preferred embodiment of the method according to the invention,
Fig. 3 is a photography of the surface of a collagen sponge produced by a method according to the invention (courtesy Prof. Dr. Roman Carbon, Chirurgische Univ. Klinik Erlangen, Germany), and
Fig. 4 discloses a stirring device for obtaining a measure of the viscosity of a collagen gel.

### DESCRIPTION OF THE DRAWINGS

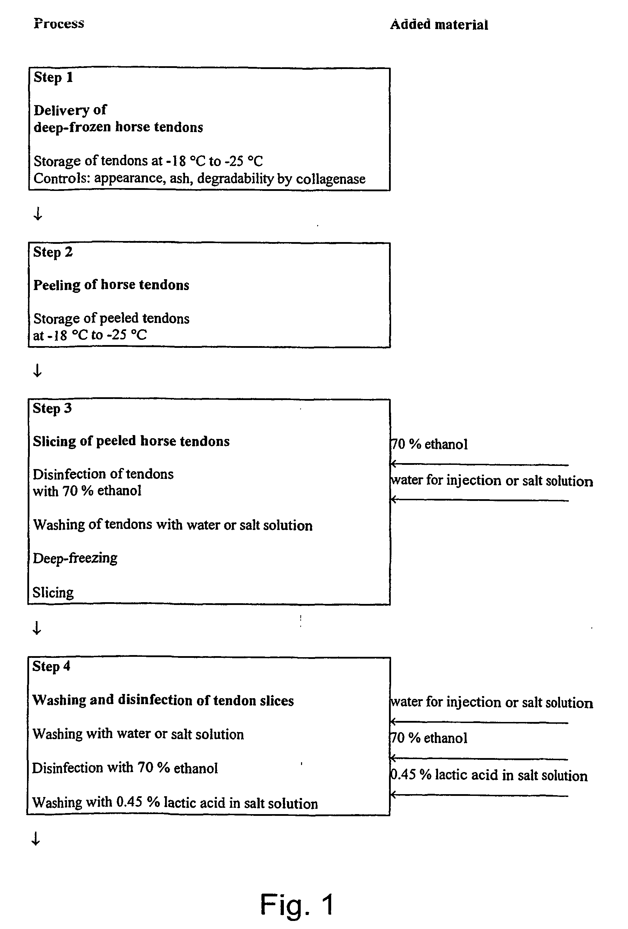

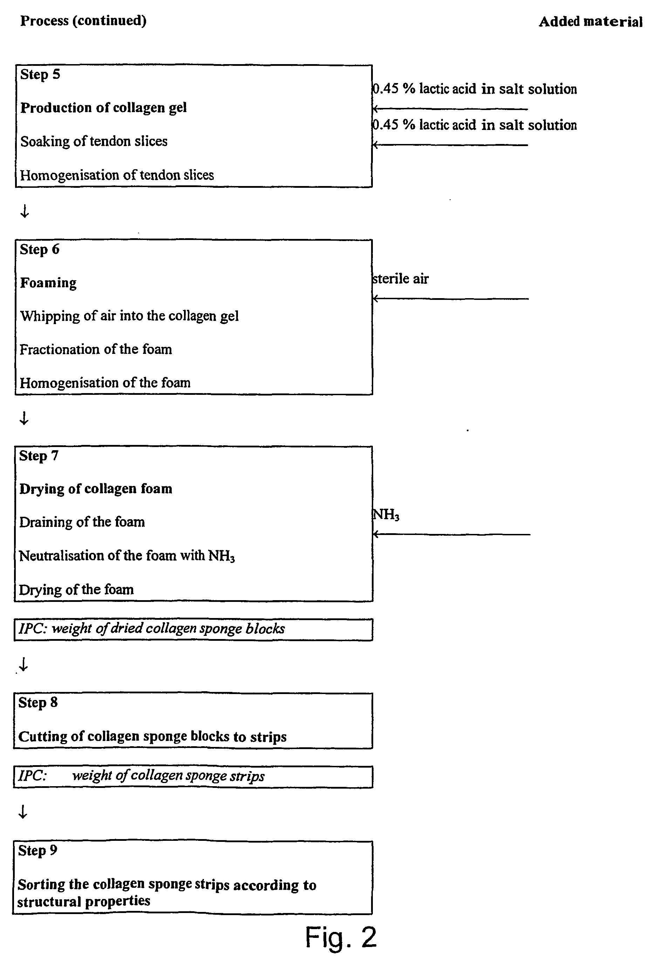

In a preferred embodiment, the invention comprises the following steps as illustrated in Figs. 1 and 2:

### Step 1 Delivery of deep-frozen horse tendons

The horse tendons are delivered and stored at -18 °C to -25 °C.

### Step 2 Peeling of horse tendons

In a half-frozen state, the thin skin of the tendons is manually or mechanically removed with a knife. The tendons are then again deep-frozen at -18 °C to -25 °C.

### Step 3 Mechanical slicing of peeled horse tendons

Optionally, peeled frozen tendons are disinfected for 30 min in 70 % ethanol and passed into production rooms under ethanol. The tendons are then washed, and after washing the tendons are compacted to blocks and deep frozen at -18 °C to -25 °C. The frozen tendon blocks are then sliced with a cutting machine with a rotating knife into slices having a thickness of approximately 1 mm.

### Step 4 Washing and disinfection of the tendon slices

In order to remove soluble proteins, the tendon slices are first soaked in water for injection for 3 - 6 hours, then washed with water for injection or demineralized water or salt solutions containing Ca²⁺ and/or Mg²⁺-ion within the range of 1-10mM until the supernatant is free of hemoglobin. The tendon slices are then disinfected in 70 % ethanol for 15 min and washed twice in 0.45 % lactic acid in drinking water (sterile filtered and depyrogenised) to remove the ethanol.

### Step 5 Production of collagen gel

The washed tendon slices are soaked in 0.45 % lactic acid for 2-5 days, preferably 4 days, and then homogenised to a collagen gel. Exposure to 0.45 % lactic acid is considered to be one of the main virus inactivation steps.

### Step 6 Foaming

With dissolver stirrers sterile filtered air is whipped into the collagen gel. The arising foam is fractioned, and the fraction with a bubble size of 1 - 3 mm is collected. The foam is poured from the steel container into a barrel which is slowly rotated for approximately 3 minutes to obtain a homogeneous foam. This foam is filled into drying containers. The base of the container consists of a textile tissue which is permeable to fluids, so as to allow draining of the foam. After 5 - 24 hours, preferably 18-24 hours, the drained foam is exposed to ammonia gas e.g generated from a 26 % ammonia solution of DAB quality. During this process, the surplus of ammonia shifts the pH of the foam to the alkaline region. Ammonia is removed during the subsequent drying process resulting in a neutral product.

### Step 7 Drying the foam

The foam is dried in warm air in a high grade steel drying chamber for 48 - 150 hours, preferably 120 - 150 hours. The result is collagen sponge shaped in blocks.

### Step 8 Cutting the collagen blocks

Blocks, also referred to as sheets, of collagen may for example be used as carriers for a coating. The cutting is performed with a vertical cutting machine. First, the sides of the block are cut off to yield a block with vertical sides with a side length of 50 cm. This block is then cut vertically into 4 bars with a width of 11 cm. The bars are again trimmed at their upper and their lower side and then sliced into strips with dimensions of 50 × 11 × 0.4 - 0.7 cm. The weight of the collagen sponge strips preferably takes into account any specification of collagen in the final product to be achieved, such as TachoComb® H, TachoComb® and Tachotop®.

### Step 9 Sorting the collagen sponge strips

The collagen sponge strips are then subjected to a visual control. Strips with one or more of the following defects are discarded:
- strips with an average chamber diameter smaller than 1 mm or larger than 3 mm
- strips with inhomogeneous chamber structure
- strips with holes (single chambers with a depth larger than the thickness of the sponge)

The sorted strips are stored for maximally 1 year in disinfected light metal containers at a temperature of 15 - 25 °C.

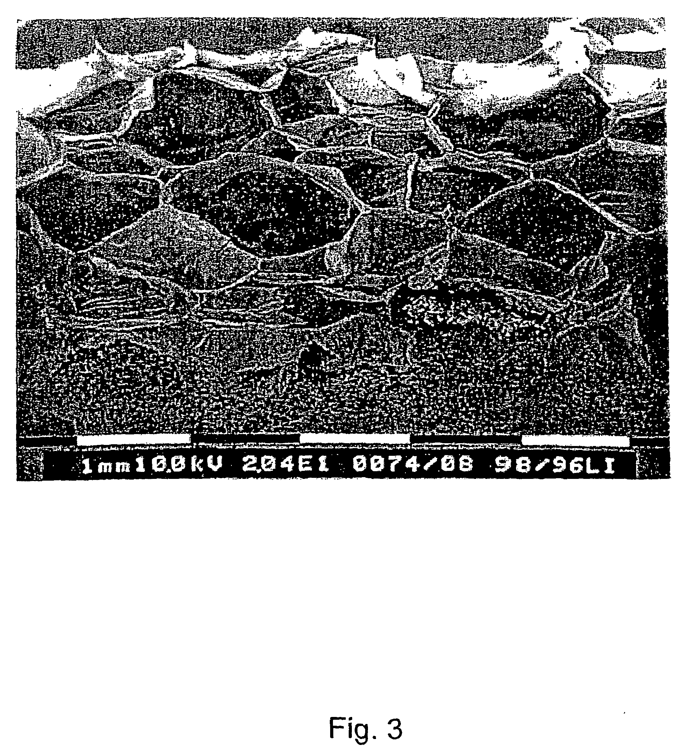

Fig. 3 is a photography of the surface of a collagen sponge produced by a method according to the invention, the photography being taken at a magnification factor of approximately 20,000 (courtesy Prof. Dr. Roman Carbon, Chirurgische Univ. Klinik Erlangen, Germany). The surface shown in the photography of Fig. 3 is a surface of a cross-sectional cut in a collagen sponge prepared by a method according to the present invention. The dark areas in the photography represent chambers, while the light areas in the photography represent collagen material, including walls of collagen material separating the chambers.

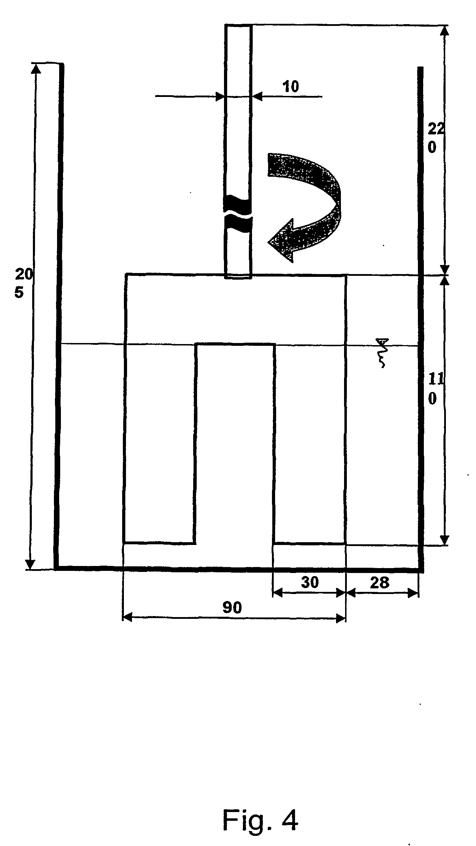

Fig. 4 shows a stirring device for obtaining a measure of a viscosity of a liquid, comprising a container adapted to accommodate a liquid and means for stirring said liquid. The stirring means comprise a rod attached to a fork shaped element. A liquid in the container is stirred by applying a torque to the rod, resulting in a rotational movement of the fork shaped element. The fork shaped element comprises a primary part 41 to which the rod is attached and a first and a second secondary part 42. The secondary parts are attached to the ends of the primary part As the fork shaped element rotates the surfaces moves the liquid and thus stirs the liquid.

In one embodiment the device has the following dimensions. The container is 110 mm high and 146 mm wide. The rod is 220 mm high and has a diameter of 10 mm. The primary part 41 of the fork is 90 mm long and 30 mm high. The secondary parts 42 are 90 mm high and 30 mm wide. The distance from an outer edge of the secondary parts 42 to an inner surface of the container is 28 mm.

### Example I

Table I below shows parameter values of three different cycles of the method according to the invention.

**Table I**

| | Cycle 1 | Cycle 2 | Cycle 3 |
|---|---|---|---|
| Peeling of tendons: waste (%) | 24 | 40 | 38 |
| Bioburden before peeling CFU/g tendon | 7x10⁴ | 2.4x10⁵ | 5x10⁵ |
| Bioburden after peeling (CFU/g tendon) | - | 2x10³ | 5x10³ |
| Tendon weight per batch | 12.00 kg | 10.5 kg | 10.5 kg |
| Washing of peeled tendons with demineralized water | 30min | 30min | 30min |
| Removal of soluble protein:washing of sliced tendons with demineralized water until washing solution is free of hemoglobin | 5h | 5h | 1.5h |
| Disinfection with 70% ethanol | 15min | 15min | 15min |
| Washing in 0.45% lactic acid | 21min | 21min | 21min |
| Soaking in 0.45% lactic acid | 144h | 120h | 120h |
| Homogenisation- | Condux tooth mill | Condux tooth mill | Condux tooth mill |
| Gel viscosity (torque) | 6.9-7.8 Ncm | 6.9-7.8 Ncm | 7.1-9.5 Ncm |
| Dry mass of collagen gel | 6.3-8.3mg/g | 8.9-9.4mg/g | 7.8-9.4mg/g |
| Foaming time per block | 37-47min | 51-58min | 54-62min |
| Bioburden (CFU/ml wet foam) | - | 2 | 1 |
| Draining period | 18.5h | 22h | 18h |
| Neutralisation period | 24h | 24h | 24.5h |
| Drying period | 147h | 148.5h | 144.5h |
| Weight per block | 200-256g | 195-228g | 177-257g |
| Bioburden of collagen sponge strips (CFU/g) | 14-1000 | < 18-124 | < 11-33 |
| Yield of collagen sponge strips: | 405 | 379 | 433 |
| Length:110mm | | | |
| Width: 500mm | | | |
| Height: 4-7mm | | | |
| Weight: 770-1500mg/strip | | | |

Table II below shows parameter values of three different collagen sponges obtained by the method according to the invention.

**Table II**

| | Sponge I | Sponge II | Sponge III |
|---|---|---|---|
| pH value (spec: 4-6) | 5.4 | 5.1 | 5.4 |
| Lactic acid content | 2.6% | 2.8% | 2% |
| Ammonium content | 0.2% | 0.2% | 0.1% |
| Soluble protein content | 0.1% | 0.05% | 0.08% |
| Sulphate ashes content | 0.4% | 0.3% | 0.3% |
| Microbiological purity (CFU/g) | 14-1000 | <18-124 | <11-33 |
| Collagen content related to dry mass | 95% | 95% | 98% |
| Water content | 14% | 15% | 16% |
| Elasticity module | 10-45 N/cm² | 15-50 N/cm² | 12.3-41.0 N/cm² |
| Chamber size (diameter; mean value) | 2.3mm | 2.1mm | 2.9mm |
| Density | 2.5-6.1mg/cm³ | 2.9-5.9mg/cm³ | 2.4-5.0mg/cm³ |

| | Sponge IV | Sponge V | |
|---|---|---|---|
| pH value | 5.3 | 5.7 | |
| Lactic acid content | 2.3% | 1.1% | |
| Ammonium content | 0.1% | 0.1% | |
| Soluble protein content | 0.04% | 0.11% | |
| Sulphate ashes content | 0.3% | 0.2% | |
| Heavy metal content | <20ppm | <20ppm | |
| Microbiological purity | <12-345CFU/g | <15-48 CFU/g | |
| Collagen content related to dry mass | 95% | 96% | |
| Water content | 14% | 12% | |
| Elasticity module | 10.4-42.1N/cm² | 20-47N/cm² | |
| Chamber size (diameter; mean value) | 2.9mm | 2.5 mm | |
| Density | 2.9-5.3mg/cm³ | 2-6.8mg/cm³ | |

### Example II

This example relates to the indirect measurement of the viscosity of the collagen gel by torque measurement.

The equipment used for the torque measurement is:
- Stirring machine: EUROSTAR POWER control-visc
- Windows-Software: IKASOFT dc
- Torque indicator: VISCOKLICK VK 1
- Datalogger DC 2
- Special stirrer construction ("fork") with defined dimensions, cf. Fig. 4
- Funnel with an inner diameter of 14.6 cm and a height of 20.5 cm
- Thermometer
- Balance

An amount of 1500 g of collagen gel is filled into the funnel. The temperature of the sample is 23°C. The "fork" stirrer is fixed in the centre of the funnel. Then measurement is started. The Torque indicator is transforming the resistance of the stirrer into a value (Ncm) representing the dynamic gel viscosity.

In order to verify the measurement of the torque, a standard solution of 59% Polyethylenglycol is prepared. Viscosity of this solution is measured by a Haake Visosimeter RV 20 Rotovisko. The dynamical viscosity η of this solution is in the range of η = 925 ± 25 mPas at 23°C. This solution viscosity is measured by the above gel measuring equipment, and the torque value thereby measured should be in the range of 3.66 Ncm ± 5% at 23°C.

### REFERENCES

1. Baer, E. Gathercole, L.J. and Keller, A., *Structure hierarchies in tendon collagen: an interim summary,* Proc. Colston Conf., 1974, 189; Hiltner, A. Cassidy, J.J, and Baer, E., *Mechanical properties of biological polymers,* Ann. Rev. Mater. Sci., 15, 455, 1985)

## Claims

1. A method of preparing a collagen sponge, comprising the steps of:
- preparing a collagen gel,
- mixing air into the collagen gel, so as to obtain a collagen foam,
- drying the collagen foam, so as to obtain a dry block of collagen sponge having a three-dimensional structure with stacked chambers which are separated and substantially totally enclosed by walls of collagen material,
- isolating, from the block of collagen sponge, parts of sponge with a chamber diameter of more than 0.75 mm and less than 4 mm, or parts with an average chamber diagonal dimension of 3 mm.

2. A method according to claim 1, wherein the collagen content of the isolated parts of sponge is 50 to 100%.

3. A method according to claim 2 or 3, wherein the collagen gel comprises collagen material of different types from at least one of the following sources: mammalian, transgenic and recombinant sources.

4. A method according to claim 3, wherein the collagen comprises material from tendons selected from the group consisting of equine tendons, bovine tendons and human tendons.

5. A method according to claim 3 or 4, wherein the step of preparing the collagen gel comprises the steps of:
- storing the tendons at a temperature between -10°C and -30°C, and peeling the tendons,
- removing foreign protein from the tendons,
- reducing germ content in the tendons,
- swelling the tendons,
- homogenising the swelled tendons.

6. A method according to claim 5, wherein the step of reducing germ content comprises adding an acid and an organic solvent to the tendons.

7. A method according to claim 6, wherein acid is an organic acid, such as lactic acid.

8. A method according to claim 6 or 7, wherein the organic solvent is an alcohol, such as ethanol.

9. A method according to any of claims 5-8, wherein the step of swelling the tendons comprises adding lactic acid to the tendons.

10. A method according to any of claims 5-9, wherein the acid has a pH value in the range of 1 to 4, such as 1.5 to 3.5, such as 2.5 to 3.0.

11. A method according to any of claims 5-10, wherein the lactic acid is a 0.45% lactic acid.

12. A method according to any of claims 5-11, wherein the step of swelling the tendons comprises storing the tendons at a temperature of 4°C to 25°C for a period of 48 to 200 hours.

13. A method according to claim 12, wherein the tendons are stored for a period of 100 to 120 hours.

14. A method according to any of claims 5-13, wherein the step of homogenising the swelled tendons comprises obtaining a substance comprising particles of tendons, the particles having a length or diameter of 0.8 to 1.2 cm.

15. A method according to any of claims 5-14, wherein the step of homogenising the swelled tendons comprises obtaining a substance having a viscosity of 2 to 20 Ncm.

16. A method according to any of claims 5-15, wherein the step of homogenising the swelled tendons is carried out by means of a toothed disk mill or comparable equipment

17. A method according to any of the preceding claims, wherein the collagen gel has a dynamic viscosity of 2-20 Ncm.

18. A method according to any of the preceding claims, wherein the step of mixing air into the collagen gel comprises the steps of:
- mixing ambient air into the gel by means of a mixer so as to generate a collagen foam,
- feeding the mixed gel foam into a fractionising channel,
- separating collagen gel and collagen foam contained in the fractionising channel.

19. A method according to claim 18, wherein at least some of the collagen gel separated from the collagen foam in the fractionising channel is led back to the mixer.

20. A method according to claim 19, wherein the ratio between the amount of collagen gel which is led back to the mixer from the fractionising channel and the amount of fresh collagen gel led to the mixer is between 0.1 and 0.5.

21. A method according to any of claims 18-20, wherein the step of separating collagen gel and collagen foam comprises the steps of:
- separating a selected part of the collagen foam contained in the fractionising channel,
- leading the selected part of the collagen foam out of the fractionising channel for drying thereof.

22. A method according to any of claims 18-21, further comprising maintaining a temperature between 15°C and 40°C in the fractionising channel.

23. A method according to any of the preceding claims, further comprising, subsequent to mixing air into the collagen gel, homogenising the collagen foam for a period of 2 to 4 minutes.

24. A method according to any of the preceding claims, further comprising, prior to the step of drying the collagen foam and subsequent to the step of mixing air into the collagen gel, adding a neutraliser to the collagen foam and neutralising the collagen foam in order to achieve a pH-value in the collagen foam between 6.5 and 8.5.

25. A method according to claim 24, wherein the neutraliser comprises an ammonia solution.

26. A method according to claim 24 or 25, wherein the collagen foam is neutralised for a period of 5-30 hours.

27. A method according to claim 26, wherein the collagen foam is neutralised for a period of 20-30 hours.

28. A method according to any of the preceding claims, wherein the step of drying comprises drying at a temperature between 15°C and 60°C for a period of 48-200 hours, so as to obtain a dry collagen sponge.

29. A method according to claim 28, wherein the step of drying is carried out at a pressure of 700 to 900 mbar.

30. A method according to any of the preceding claims, wherein the step of drying comprises drying at a temperature between 15°C and 40°C for a period of 100-200 hours.

31. A method according to any of the preceding claims, wherein the collagen sponge fulfils at least one of the following criteria:
- pH-value between 5.0 and 6.0,
- lactic acid content at the most 5%,
- ammonium content at the most 0.5%,
- soluble protein content, calculated as albumin content, at the most 0.5%,
- sulphate ashes content at the most 1.0%,
- heavy metal content at the most 20 ppm,
- microbiological purity, at the most 10³ CFU/g,
- collagen content of 75 to 100%,
- density of 1 to 10 mg/cm³,
- elasticity module in the range of 5-100 N/cm².

32. A method according to any of the preceding claims, wherein the collagen sponge has a water content of not more than 20%.

33. A method according to any of the preceding claims, wherein the step of isolating parts of collagen sponge comprises dividing the collagen sponge into a plurality of parts by cutting.

34. A method of preparing a collagen sponge, comprising the steps of:
- preparing a collagen gel,
- mixing air into the collagen gel, so as to obtain a collagen foam,
- drying the collagen foam, so as to obtain a dry block of collagen sponge having a three-dimensional structure with stacked chambers which are separated and substantially totally enclosed by walls of collagen material,
- isolating, from the block of collagen sponge, parts of sponge having the following properties:
- elasticity module in the range of 5 to 100 N/cm²,
- density in the range of 1 to 10 mg/cm³,
- chamber diameter of more than 0.75 mm and less than 4 mm, or a chamber diameter average of at most 3 mm.

35. A method according to claim 34, comprising the steps of any of claims 1-33.

## Patentansprüche

1. Verfahren zur Herstellung eines Kollagenschwammes, folgende Schritte umfassend:
- Herstellen eines Kollagengels,
- Einmischen von Luft in das Kollagengel, so dass ein Kollagenschaum erhalten wird,
- Trocknen des Kollagenschaums, so dass ein trockener Block aus Kollagenschwamm erhalten wird, der eine dreidimensionale Struktur mit gestapelten Kammern hat, die getrennt sind und im Wesentlichen völlig eingeschlossen durch Wände aus Kollagenmaterial,
- aus dem Block aus Kollagenschwamm Teile des Schwammes isolieren, mit einem Kammerdurchmesser von mehr als 0,75 mm und weniger als 4 mm oder Teile mit einer mittleren Kammerdiagonalenabmessung von 3 mm.

2. Verfahren nach Anspruch 1, wobei der Kollagengehalt der isolierten Teile des Schwammes 50 bis 100 % ist.

3. Verfahren nach Anspruch 2 oder 3, wobei das Kollagengel Kollagenmaterial verschiedener Typen umfasst, aus mindestens einer der folgenden Quellen: Säugetier-, transgene und rekombinante Quellen.

4. Verfahren nach Anspruch 3, wobei das Kollagen umfasst: Material aus Sehnen, ausgewählt aus der Gruppe, die aus Pferdesehnen, Rindersehnen und menschlichen Sehnen besteht.

5. Verfahren nach Anspruch 3 oder 4, wobei der Schritt der Herstellung des Kollagengels die Schritte umfasst:
- Lagern der Sehnen bei einer Temperatur zwischen -10 °C und -30 °C und Schälen der Sehnen,
- Entfernen von Fremdprotein von den Sehnen,
- Verringern des Keimgehaltes in den Sehnen,
- Quellen der Sehnen,
- Homogenisieren der gequollenen Sehnen.

6. Verfahren nach Anspruch 5, wobei der Schritt des Verringerns des Keimgehaltes umfasst: Zugeben einer Säure und eines organischen Lösungsmittels zu den Sehnen.

7. Verfahren nach Anspruch 6, wobei die Säure eine organische Säure wie Milchsäure ist.

8. Verfahren nach Anspruch 6 oder 7, wobei das organische Lösungsmittel ein Alkohol wie Ethanol ist.

9. Verfahren nach einem der Ansprüche 5-8, wobei der Schritt der Quellung der Sehnen das Zufügen von Milchsäure zu den Sehnen umfasst.

10. Verfahren nach einem der Ansprüche 5-9, wobei die Säure einen pH-Wert im Bereich von 1 bis 4 hat, wie 1,5 bis 3,5, wie 2,5 bis 3,0.

11. Verfahren nach einem der Ansprüche 5-10, wobei die Milchsäure eine 0,45 prozentige Milchsäure ist.

12. Verfahren nach einem der Ansprüche 5-11, wobei der Schritt der Quellung der Sehnen die Lagerung der Sehnen bei einer Temperatur von 4 °C bis 25 °C für eine Zeitdauer von 48 bis 200 Stunden umfasst.

13. Verfahren nach Anspruch 12, wobei die Sehnen für eine Zeitdauer von 100 bis 200 Stunden gelagert werden.

14. Verfahren nach einem der Ansprüche 5-13, wobei der Schritt des Homogenisierens der gequollenen Sehnen umfasst: Erhalten einer Substanz, die Partikel der Sehnen umfasst,wobei die Partikel eine Länge oder einen Durchmesser von 0,8 bis 1,2 cm haben.

15. Verfahren nach einem der Ansprüche 5-14, wobei der Schritt des Homogenisierens der gequollenen Sehnen umfasst: Erhalten einer Substanz, die eine Viskosität von 2 bis 20 Ncm hat.

16. Verfahren nach einem der Ansprüche 5-15, wobei der Schritt des Homogenisierens der gequollenen Sehnen mittels einer Zahnscheibenmühle oder einer vergleichbaren Vorrichtung durchgeführt wird.

17. Verfahren nach einem der vorhergehenden Ansprüche, wobei das Kollagengel eine dynamische Viskosität von 2-20 Ncm hat.

18. Verfahren nach einem der vorhergehenden Ansprüche, wobei der Schritt des Einmischens der Luft in das Kollagengel die Schritte umfasst:
- Einmischen von Umgebungsluft in das Gel mittels eines Mischers, so dass ein Kollagenschaum erzeugt wird,
- Zuführen des gemischten Gelschaums in einen Trennkanal,
- Trennen von Kollagengel und Kollagenschaum, die in dem Trennkanal enthalten sind.

19. Verfahren nach Anspruch 18, wobei mindestens etwas Kollagengel, getrennt vom Kollagenschaum im Trennkanal, in den Mischer zurückgeleitet wird.

20. Verfahren nach Anspruch 19, wobei das Verhältnis zwischen der Menge des Kollagengels, das aus dem Trennkanal in den Mischer zurückgeleitet wird, und der Menge an frischem Kollagengel, das in den Mischer geleitet wird, zwischen 0,1 und 0,5 ist.

21. Verfahren, nach einem der Ansprüche 18-20, wobei der Schritt des Trennens von Kollagengel und Kollagenschaum die Schritte umfasst:
- Abtrennen eines ausgewählten Teil des Kollagenschaums, der im Trennkanal enthalten ist,
- Leiten des ausgewählten Teils des Kollagenschaums aus dem Trennkanal, um ihn zu trocknen.

22. Verfahren nach einem der Ansprüche 18-21, weiterhin umfassend: Aufrechterhalten einer Temperatur zwischen 15 °C und 40 °C im Trennkanal.

23. Verfahren nach einem der vorhergehenden Ansprüche, weiterhin umfassend: nachfolgend zum Einmischen der Luft in das Kollagengel, Homogenisieren des Kollagenschaums für eine Zeitdauer von 2 bis 4 Minuten.

24. Verfahren nach einem der vorhergehenden Ansprüche, weiterhin umfassend: vor dem Schritt des Trocknens des Kollagenschaums und nachfolgend dem Schritt des Einmischens von Luft in das Kollagengel: Zugeben eines Neutralisationsmittels zu dem Kollagenschaum und Neutralisieren des Kollagenschaums, um einen pH-Wert in dem Kollagenschaum zwischen 6,5 und 8,5 zu erreichen.

25. Verfahren nach Anspruch 24, wobei das Neutralisationsmittel eine Ammoniaklösung umfasst.

26. Verfahren nach Anspruch 24 oder 25, wobei der Kollagenschaum für eine Zeitdauer von 5-30 Stunden neutralisiert wird.

27. Verfahren nach Anspruch 26, wobei der Kollagenschaum für eine Zeitdauer von 20-30 Stunden neutralisiert wird.

28. Verfahren nach einem der vorhergehenden Ansprüche, wobei der Schritt des Trocknens umfasst: Trocknen bei einer Temperatur zwischen 15 °C und 60 °C für eine Zeitdauer von 48-200 Stunden, so dass ein trockener Kollagenschwamm erhalten wird.

29. Verfahren nach Anspruch 28, wobei der Schritt des Trocknens bei einem Druck von 700 bis 900 mbar ausgeführt wird.

30. Verfahren nach einem der vorhergehenden Ansprüche, wobei der Schritt des Trocknens umfasst: Trocknen bei einer Temperatur zwischen 15 °C und 40 °C für eine Zeitdauer von 100-200 Stunden.

31. Verfahren nach einem der vorhergehenden Ansprüche, wobei der Kollagenschwamm mindestens eines der folgenden Kriterien erfüllt:
- pH-Wert zwischen 5,0 und 6,0,
- Milchsäuregehalt höchstens 5 %,
- Ammoniumgehalt höchstens 0,5 %,
- Gehalt an löslichem Protein, berechnet als Albumingehalt, höchstens 0,5 %,
- Sulfatasche-Gehalt höchstens 1,0 %,
- Schwermetallgehalt höchstens 20 ppm,
- mikrobiologische Reinheit höchstens 10³KBE/g,
- Kollagengehalt von 75 bis 100 %,
- Dichte von 1 bis 10 mg/cm³,
- Elastizitätsmodul im Bereich von 5-100 N/cm².

32. Verfahren nach einem der vorhergehenden Ansprüche, wobei der Kollagenschwamm einen Wassergehalt von nicht mehr als 20 % hat.

33. Verfahren nach einem der vorhergehenden Ansprüche, wobei der Schritt des Isolierens von Teilen des Kollagenschwammes umfasst: Teilen des Kollagenschwammes in eine Vielzahl von Teilen durch Schneiden.

34. Verfahren zum Herstellen eines Kollagenschwamms, umfassend die Schritte:
- Herstellen eines Kollagengels,
- Einmischen von Luft in das Kollagengel, so dass ein Kollagenschaum erhalten wird,
- Trocknen des Kollagenschaums, so dass ein trockener Block aus Kollagenschwamm erhalten wird, der eine dreidimensionale Struktur mit gestapelten Kammern hat, die getrennt sind und im Wesentlichen völlig von Wänden aus Kollagenmaterial eingeschlossen sind,
- aus dem Block aus Kollagenschwamm isolieren von Teilen des Schwamms, die folgende Eigenschaften haben:
- Elastizitätsmodul im Bereich 5 bis 100 N/cm²,
- Dichte im Bereich von 1 bis 10 mg/cm³,
- Kammerdurchmesser von mehr als 0,75 mm und weniger als 4 mm oder einen Kammerdurchmesserdurchschnitt von höchstens 3 mm.

35. Verfahren nach Anspruch 34, umfassend die Schritte von einem der Ansprüche 1-33.

## Revendications

1. Procédé de préparation d'une éponge de collagène, comprenant les étapes où :
- on prépare un gel de collagène,
- on mélange de l'air dans le gel de collagène, de manière à obtenir une mousse de collagène,
- on séche de la mousse de collagène, de manière à obtenir un bloc sec d'éponge de collagène possédant une structure tridimensionnelle avec des cavités superposées, qui sont séparées et essentiellement renfermées dans des parois de matière collagène,
- on isole, à partir du bloc d'éponge de collagène, des fractions d'éponge possédant un diamètre de cavité supérieur à 0,75 mm et inférieur à 4 mm, ou des fractions possédant une dimension diagonale moyenne de cavité de 3 mm.

2. Procédé selon la revendication 1, dans lequel la teneur en collagène des fractions isolées de l'éponge est de 50 à 100 %.

3. Procédé selon la revendication 2 ou 3, dans lequel le gel de collagène comprend une matière collagène de différents types issue d'au moins une source parmi les sources suivantes : source mammifère, transgénique et recombinante.

4. Procédé selon la revendication 3, dans lequel le collagène comprend des matières provenant de tendons, sélectionnés dans le groupe consistant en les tendons équins, les tendons bovins et les tendons humains.

5. Procédé selon la revendication 3 ou 4, dans lequel l'étape de préparation du gel de collagène comprend les étapes de :
- conservation des tendons à une température comprise entre -10 °C et -30 °C et détachement des tendons,
- retrait des protéines étrangères des tendons,
- réduction de la teneur en germes des tendons,
- gonflement des tendons,
- homogénéisation des tendons gonflés.

6. Procédé selon la revendication 5, dans lequel l'étape de réduction de la teneur en germes comprend l'ajout d'un acide et d'un solvant organique aux tendons.

7. Procédé selon la revendication 6, dans lequel l'acide est un acide organique, tel que l'acide lactique.

8. Procédé selon la revendication 6 ou 7, dans lequel le solvant organique est un alcool, tel que l'éthanol.

9. Procédé selon l'une quelconque des revendications 5 à 8, dans lequel l'étape de gonflement des tendons comprend l'ajout d'acide lactique aux tendons.

10. Procédé selon l'une quelconque des revendications 5 à 9, dans lequel l'acide possède une valeur de pH située dans la plage allant de 1 à 4, telle que 1,5 à 3,5, telle que 2,5 à 3,0.

11. Procédé selon l'une quelconque des revendications 5 à 10, dans lequel l'acide lactique est de l'acide lactique à 0,45 %.

12. Procédé selon l'une quelconque des revendications 5 à 11, dans lequel l'étape de gonflement des tendons comprend la conservation des tendons à une température de 4 °C à 25 °C pendant une période de 48 à 200 heures.

13. Procédé selon la revendication 12, dans lequel les tendons sont conservés pendant une période de 100 à 120 heures.

14. Procédé selon l'une quelconque des revendications 5 à 13, dans lequel l'étape d'homogénéisation des tendons gonflés comprend l'obtention d'une substance comprenant des particules de tendons, les particules possédant une longueur ou un diamètre de 0,8 à 1,2 cm.

15. Procédé selon l'une quelconque des revendications 5 à 14, dans lequel l'étape d'homogénéisation des tendons gonflés comprend l'obtention d'une substance possédant une viscosité de 2 à 20 Ncm.

16. Procédé selon l'une quelconque des revendications 5 à 15, dans lequel l'étape d'homogénéisation des tendons gonflés est réalisée au moyen d'un broyeur à disques dentelés ou d'un équipement comparable.

17. Procédé selon l'une quelconque des revendications précédentes, dans lequel le gel de collagène possède une viscosité dynamique de 2 à 20 Ncm.

18. Procédé selon l'une quelconque des revendications précédentes, dans lequel l'étape de mélange d'air dans le gel de collagène comprend les étapes de
- mélange d'air ambiant dans le gel au moyen d'un mélangeur, de manière à générer une mousse de collagène,
- introduction de la mousse de collagène mixée dans un canal de fractionnement,
- séparation du gel de collagène et de la mousse de collagène contenus dans le canal de fractionnement.

19. Procédé selon la revendication 18, dans lequel au moins une partie du gel de collagène séparé de la mousse de collagène dans le canal de fractionnement est réintroduite dans le mélangeur.

20. Procédé selon la revendication 19, dans lequel le rapport entre la quantité de gel de collagène ramené dans le mélangeur depuis le canal de fractionnement à la quantité de gel de collagène frais amené dans le mélangeur est compris entre 0,1 et 0,5.

21. Procédé selon l'une quelconque des revendications 18 à 20, dans lequel l'étape de séparation du gel de collagène et de la mousse de collagène comprend les étapes où :
- on sépare une fraction choisie de la mousse de collagène contenue dans le canal de fractionnement,
- on conduit la fraction sélectionnée de la mousse de collagène hors du canal de fractionnement pour son séchage.

22. Procédé selon l'une quelconque des revendications 18 à 21, comprenant en outre le maintien d'une température comprise entre 15 °C et 40 °C dans le canal de fractionnement.

23. Procédé selon l'une quelconque des revendications précédentes, comprenant en outre, après le mélange d'air dans le gel de collagène, une homogénéisation de la mousse de collagène pendant une période de 2 à 4 minutes.

24. Procédé selon l'une quelconque des revendications précédentes, comprenant en outre, avant l'étape de séchage de la mousse de collagène et après l'étape de mélange d'air dans le gel de collagène, l'ajout d'un agent neutralisant à la mousse de collagène et la neutralisation de la mousse de collagène de manière à obtenir une valeur de pH dans la mousse de collagène comprise entre 6,5 et 8,5.

25. Procédé selon la revendication 24, dans lequel l'agent neutralisant comprend une solution d'ammoniaque.

26. Procédé selon la revendication 24 ou 25, dans lequel la mousse de collagène est neutralisée pendant une période de 5 à 30 heures.

27. Procédé selon la revendication 26, dans lequel la mousse de collagène est neutralisée pendant une période de 20 à 30 heures.

28. Procédé selon l'une quelconque des revendications précédentes, dans lequel l'étape de séchage comprend le séchage à une température comprise entre 15 °C et 60 °C pendant une période de 48 à 200 heures, de manière à obtenir une éponge de collagène sèche.

29. Procédé selon la revendication 28, dans lequel l'étape de séchage est conduite à une pression de 700 à 900 mbar.

30. Procédé selon l'une quelconque des revendications précédentes, dans lequel l'étape de séchage comprend le séchage à une température comprise entre 15 °C et 40 °C pendant une période de 100 à 200 heures.

31. Procédé selon l'une quelconque des revendications précédentes, dans lequel l'éponge de collagène remplit au moins l'un des critères suivants :
- valeur de pH comprise entre 5,0 et 6,0,
- teneur en acide lactique de 5 % au maximum,
- teneur en ammoniaque de 0,5 % au maximum,
- teneur en protéine soluble, calculée sous forme de teneur en albumine, de 0,5 % au maximum,
- teneur en cendres de sulfate de 1,0 % au maximum,
- teneur en métaux lourds de 20 ppm au maximum,
- pureté microbiologique de 10³ UFC/g au maximum,
- teneur en collagène de 75 à 100 %,
- densité de 1 à 10 mg/cm³,
- module d'élasticité dans la plage de 5 à 100 N/cm².

32. Procédé selon l'une quelconque des revendications précédentes, dans lequel l'éponge de collagène possède une teneur en eau ne dépassant pas 20%.

33. Procédé selon l'une quelconque des revendications précédentes, dans lequel l'étape d'isolement de fractions d'éponge de collagène comprend la division de l'éponge de collagène en plusieurs fractions par découpe.

34. Procédé de préparation d'une éponge de collagène, comprenant les étapes où:
- on prépare un gel de collagène,
- on mélange de l'air dans le gel de collagène, de manière à obtenir une mousse de collagène,
- on sèche de la mousse de collagène, de manière à obtenir un bloc sec d'éponge de collagène possédant une structure tridimensionnelle avec des cavités superposées, qui sont séparées et essentiellement renfermées dans des parois de matière collagène,
- on isole du bloc d'éponge de collagène, de fractions d'éponge possédant les propriétés suivantes :
- module d'élasticité dans la plage de 5 à 100 N/cm².
- densité dans la plage de 1 à 10 mg/cm³,
- diamètre de la cavité supérieur à 0,75 mm et inférieur 4 mm, ou diamètre moyen de la cavité de 3 mm au maximum.

35. Procédé selon la revendication 34, comprenant les étapes de l'une quelconque des revendications 1 à 33.
